# EUROPEAN PATENT SPECIFICATION

(11) **EP 2 016 969 B1**
(45) Date of publication and mention of the grant of the patent: **28.04.2010**
(21) Application number: 08012493.6
(22) Date of filing: 10.07.2008
(51) Int. Cl.: A61M 25/00

(54) **Guiding catheter**
Führungskatheter
Cathéter de guidage

(30) Priority: 18.07.2007 JP 2007186978
(43) Date of publication of application: 21.01.2009
(73) Proprietor: Nipro Corporation, Kita-ku, Osaka-shi, Osaka-fu, 531-8510 (JP)
(72) Inventor: Koyama, Yutaka, Kanagawa-ken, 234-0055 (JP); Tanikawa, Masahiro c/o Nipro Corporation, Osaka-fu, 531-8510 (JP)
(74) Representative: Sticht, Andreas

(56) References cited:
- WO-A-97/02066
- WO-A-97/26038
- US-A- 5 348 545

## Description

### Technical Field

The present invention relates to a guiding catheter for a right coronary artery which is easily inserted into a coronary artery by a trans-radial coronary intervention and hardly comes off from the coronary artery owing to a strong pushing force.

Such a guiding catheter is disclosed in the WO 9702066.

### Related Art

In the past, a guiding catheter for a coronary artery has been used so as to guide a therapeutic device into the coronary artery. In addition, in a Percutaneous Coronary Intervention (PCI), in order to enlarge a narrowed portion in the coronary artery of a heart, a guide wire for a guiding catheter is first inserted into an artery blood vessel in terms of a Seldinger technique or the like. Subsequently, the guiding catheter is inserted into an artery blood vessel along the guide wire so that the distal end portion is positioned at a coronary artery inlet of a heart.

Thereafter, only the guide wire is taken out therefrom and then, a distal tip of the guiding catheter is inserted into the coronary artery. Subsequently, a thin guide wire for a therapeutic device thinner than the guiding catheter is inserted along the guiding catheter so that the distal end portion arrives at a position passed through a narrowed portion. Subsequently, a distal end portion of a therapeutic device such as a balloon catheter is inserted into a proximal end portion of the guide wire, the therapeutic device is inserted into the guiding catheter along the guide wire as a shaft, and then, the distal end portion of the therapeutic device advances in the narrowed portion.

The guiding catheter includes a main body, a distal end portion, and a curved portion provided between the main body and the distal end portion. The curved portion causes the guiding catheter to correspond to a shape of a blood vessel of the coronary artery. For instance, the curved portion may be formed into exemplary shapes such as Judkins or Amplatz or may be formed into other numerous shapes for the left and right coronary arteries.

Incidentally, in the PCI, although the therapeutic device is applied with a repulsive force occurring upon advancing a distal end portion of the therapeutic device such as the balloon catheter in the narrowed portion in the coronary artery, the guiding catheter withstands the repulsive force by allowing its curved portion to come into contact with an inner surface of an ascending aorta.

However, the repulsive force becomes large when a gap between the narrowed portion and the guide wire for the therapeutic device is small due to stenosis of the narrowed portion in the coronary artery or when the narrowed portion is stiff. As a result, the curved portion is straightened by the repulsive force, and a distal end portion of the guiding catheter may come off from the coronary artery inlet in some cases.

In this case, since the guiding catheter cannot withstand the repulsive force, it is difficult to advance the distal end portion of the therapeutic device in the narrowed portion. Such a problem may be solved by increasing the wall thickness of the guiding catheter.

However, in the recent PCI, a trans-radial coronary intervention (TRI) which relieves a patient's burden after a treatment is more frequently carried out than a trans-femoral artery intervention (TFI) which has been generally carried out so far. In this case, since a diameter of a blood vessel of a radial artery is smaller than that of a femoral artery, the TRI uses a guiding catheter with a small outer diameter.

In such a guiding catheter having a small outer diameter, sometimes, it is necessary to insert a therapeutic device having a comparatively large outer diameter thereinto or to simultaneously insert two balloon catheters thereinto. Accordingly, there has been demanded a guiding catheter for solving the selection limitation in a treatment. For this reason, the guiding catheter having a small outer diameter needs to have a large inner diameter, and thus, a problem arises in that it is difficult to increase the wall-thickness of the guiding catheter.

Therefore, in order to solve the problem that the distal end portion of the guiding catheter comes off from the coronary artery inlet because the curved portion of the guiding catheter is straightened by a large repulsive force, a tubular catheter is especially configured for inserting into the coronary artery by using a technique of inserting into a radius, the tubular catheter including: a curved portion provided at a distal end portion thereof, wherein a shape of the curved portion is formed by a first curved portion, a second curved portion, and a linear portion provided between them so as to be easily inserted into the coronary artery and to hardly come off therefrom (see, for instance, Japanese Patent Laid-Open No. 3078261).

Additionally, it is disclosed that a catheter includes a first curved portion; a first substantial linear portion; a second curved portion; a second substantial linear portion; a third curved portion; a third substantial linear portion; a fourth curved portion; and a substantial linear distal end portion, wherein when the distal end portion of the catheter is positioned at a lumen part of the left coronary artery, the first curved portion is shaped so as to be introduced into an aortic arch from a brachiocephalic artery and the second substantial linear portion is shaped so as to come into contact with an aortic arch wall on the side opposite to the lumen of the left coronary artery (see, for instance, Japanese Patent Laid-Open No. 3659502).

### Disclosure of the Invention

### Problem to be solved by the Invention

When the PCI into the right coronary artery is carried out via the trans-radial coronary intervention, a general right Judkins guiding catheter cannot satisfactorily withstand the repulsive force occurring upon advancing the therapeutic device such as the balloon catheter in the narrowed portion in which a gap between the guide wired and the narrowed portion is small due to strength of the narrowed portion, and thus, a problem arises in that it is difficult to insert the therapeutic device into the narrowed portion.

When the narrowed portion is stiff, the repulsive force becomes large, and thus, a problem arises in that it is difficult to insert the therapeutic device into the narrowed portion. When a comparatively large therapeutic device needs to be inserted into the guiding catheter during a treatment, it is necessary to use a thin guiding catheter having a small outer diameter and a large inner diameter. However, the thin guiding catheter cannot satisfactorily withstand the repulsive force, and thus, a problem arises in that it is difficult to insert the therapeutic device into the narrowed portion.

### Summary of the Invention

The present invention solves the above-described problems, and an object of the invention is to provide a guiding catheter for a coronary artery which is easily inserted into the right coronary artery even via a trans-radial coronary intervention using a small-diameter guiding catheter and which hardly comes off from the coronary artery thanks to a strong pushing force.

This object is achieved by a guiding catheter as defined in claim 1. The dependent claims define further embodiments.

In order to attain the above-described object, Aspect 1 of the invention provides a guiding catheter including: a distal end portion; a main body; and a curved portion which is provided between the distal end portion and the main body, wherein the curved portion includes a first curved portion on a distal end portion side and a second curved portion on a proximal end portion side, and wherein the first curved portion and the second curved portion are continuously curved in the same direction.

According to the guiding catheter with the configuration described in Aspect 1, it is possible to obtain the guiding catheter of which the inserted distal end portion hardly comes off therefrom owing to an excellent back-up force obtained by forming the curved portion with a shape having two different curvature radiuses so as to correspond to a lumen shape of a part into which the guiding catheter is inserted.

Aspect 2 of the invention provides the guiding catheter wherein a curvature radius of the second curved portion is larger than that of the first curved portion.

According to the guiding catheter with the configuration described in Aspect 2, it is possible to allow the main body of the guiding catheter to come into contact with a lumen wall opposed to a part, into which the distal end portion is inserted, in terms of the second curved portion having a predetermined appropriate curvature radius. In addition, it is possible to insert the distal end portion of the guiding catheter into the part, into which the distal end portion is inserted, by a predetermined degree so as not to come off therefrom in terms of the first curved portion having a predetermined appropriate curvature radius.

The invention provides the guiding catheter wherein the curvature radius of the first curved portion is in the range of 5 to 20 mm and the curvature radius of the second curved portion is in the range of 30 to 100 mm.

According to the guiding catheter with the configuration described, it is possible to form the guiding catheter for the coronary artery which has the curved portion having a size and shape insertable into the left and right coronary arteries through an ascending aorta in a human heart and can carry out a Percutaneous Coronary Intervention (PCI).

Aspect 3 of the invention provides the guiding catheter wherein a portion from the first curved portion to the distal end portion is formed into a linear shape.

According to the guiding catheter with the configuration described in Aspect 3, since a therapeutic device such as a balloon catheter can be advanced from the linear distal end portion in a linear shape, it is possible to accurately insert the therapeutic device into a predetermined diseased part.

Aspect 4 of the invention provides the guiding catheter wherein an elastic distal tip is provided at the end of the distal end portion, and wherein the distal tip is inserted from one of left and right radial arteries to a right coronary artery so that a part of the curved portion comes into contact with an aortic wall using the second curved portion having a predetermined curvature radius and the distal tip positioned at the linear distal end portion is latched in the right coronary artery using the first curved portion having a predetermined curvature radius so as not to come off from the right coronary artery.

According to the guiding catheter with the configuration described in Aspect 4, it is possible to obtain the guiding catheter for the coronary artery which hardly comes off from the coronary artery while the distal end portion is inserted into the coronary artery owing to a strong pushing force.

According to the invention, since the curved portion provided between the distal end portion and the main body includes the first curved portion on the distal end portion side and the second curved portion on the proximal end portion side which are continuously curved in the same direction, it is possible to obtain the guiding catheter of which the inserted distal end portion hardly comes off therefrom owing to an excellent back-up force obtained by forming the curved portion with a shape having two different curvature radiuses so as to correspond to a lumen shape of a part into which the guiding catheter is inserted.

### Brief Description of the Drawings

Fig. 1 is a view entirely illustrating an exemplary guiding catheter according to the invention.
Fig. 2 is a view illustrating a PCI using the guiding catheter according to the invention.
Figs. 3A and 3B are schematic views illustrating a state that the guiding catheter according to the invention is inserted into a coronary artery, where Fig. 3A is a schematic view illustrating a state that the guiding catheter is inserted into a right coronary artery via a right radial artery approach and Fig. 3B is a schematic view illustrating a state that the guiding catheter is inserted into the right coronary artery via a left radial artery approach.
Figs. 4A and 4B are explanatory views illustrating a shaft main body of the guiding catheter according to the invention, where Fig. 4A is a sectional view when viewed in an axial direction and Fig. 4B is a sectional view taken along the line E-E.

### Embodiments of the Invention

Hereinafter, a guiding catheter according to an embodiment of the invention will be described in detail with reference to the accompanying drawings. In addition, it should be understood that the embodiment is exemplary of the invention and the guiding catheter for a coronary artery according to the invention is not limited thereto.

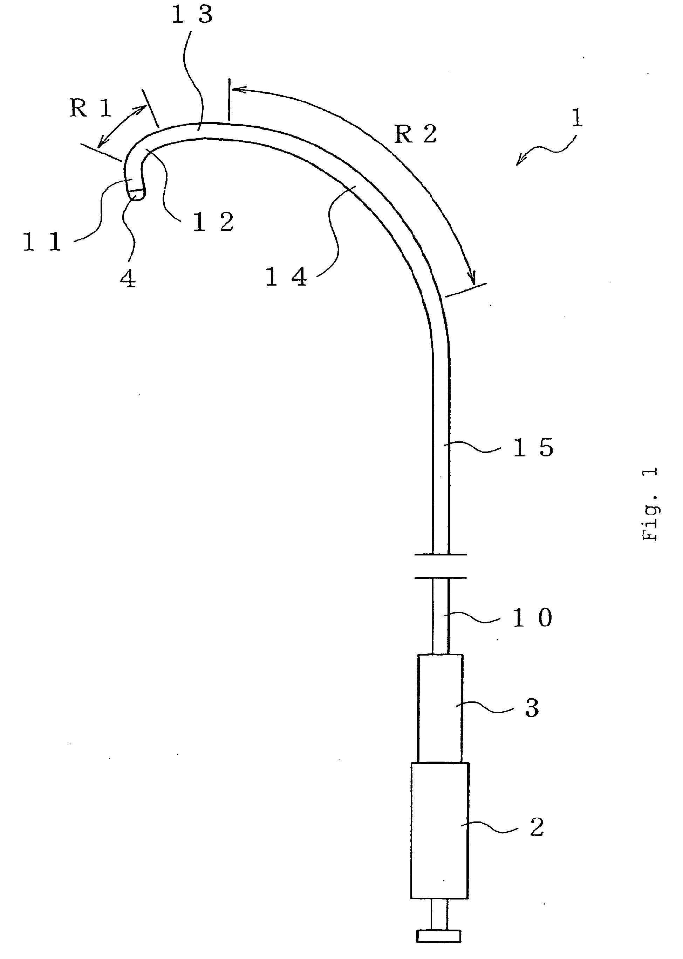

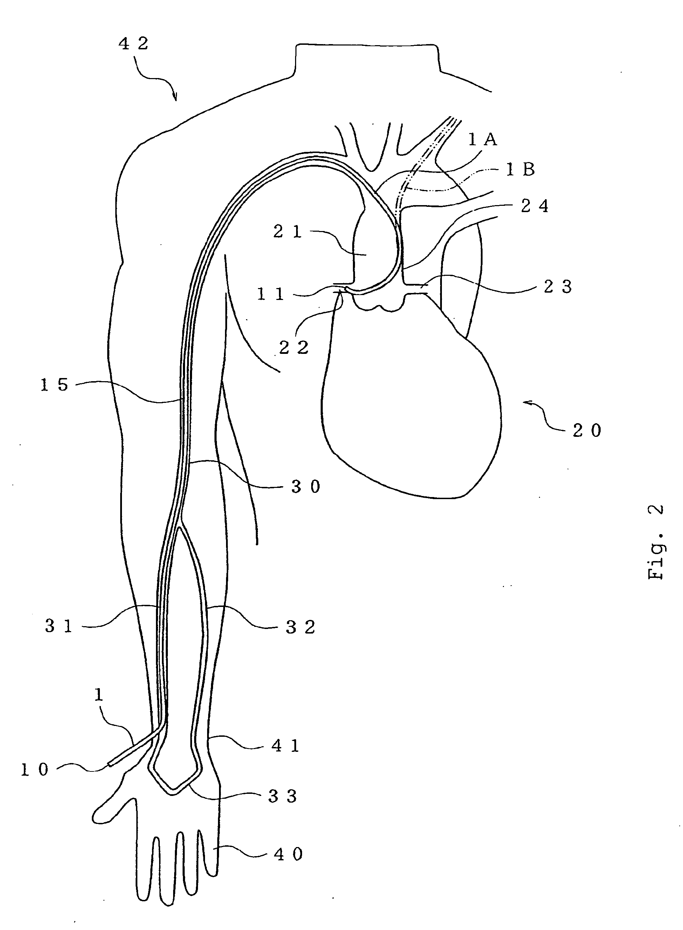

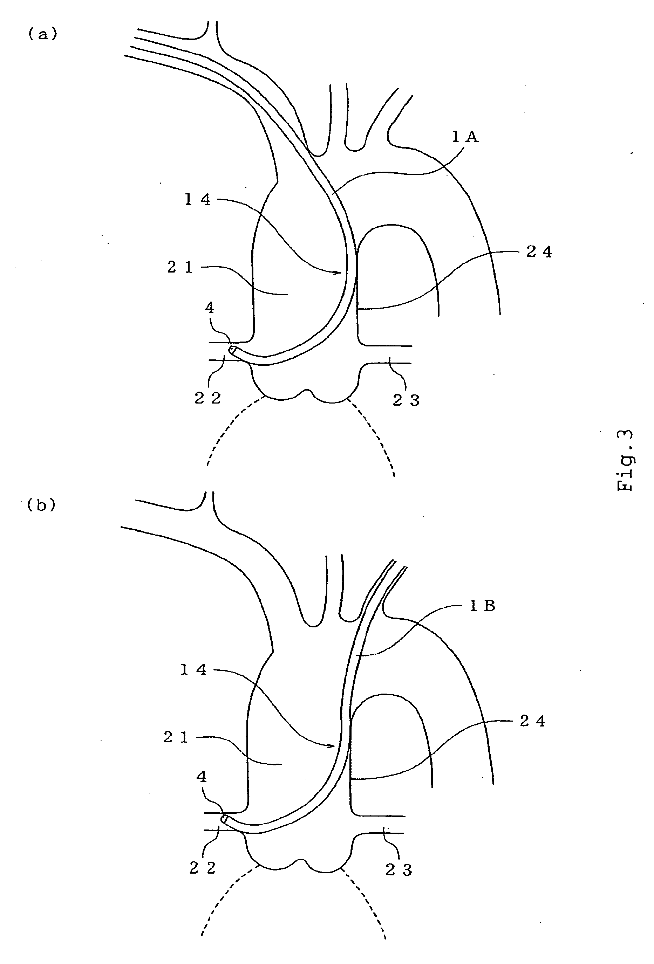

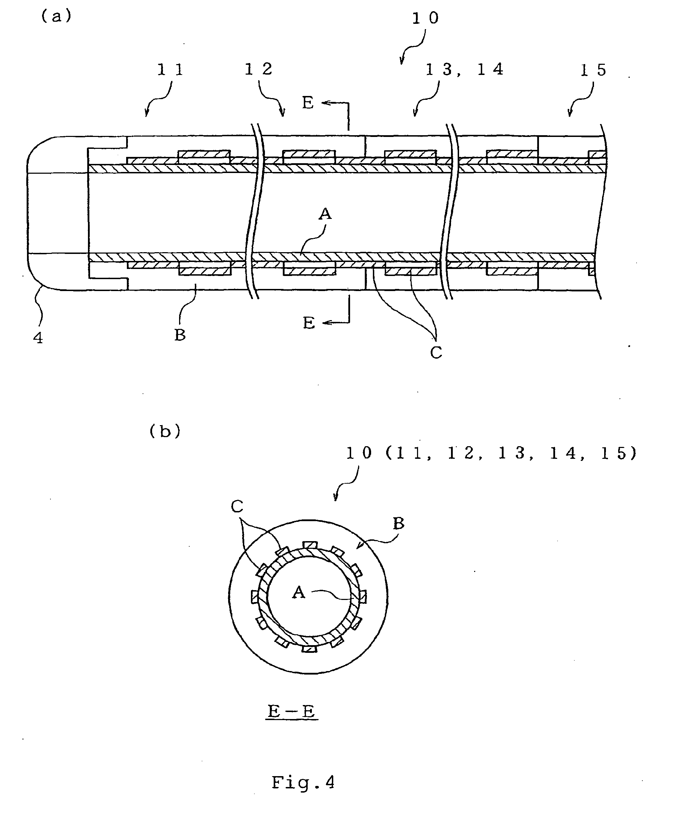

Fig. 1 is a view entirely illustrating the exemplary guiding catheter according to the invention. Fig. 2 is a view illustrating a PCI using the guiding catheter according to the invention. Figs. 3A and 3B are schematic views illustrating a state that the guiding catheter according to the invention is inserted into a coronary artery, where Fig. 3A is a schematic view illustrating a state that the guiding catheter is inserted into a right coronary artery via a right radial artery approach and Fig. 3B is a schematic view illustrating a state that the guiding catheter is inserted into the right coronary artery via a left radial artery approach. Figs. 4A and 4B are explanatory views illustrating a shaft main body of the guiding catheter according to the invention, where Fig. 4A is a sectional view when viewed in an axial direction and Fig. 4B is a sectional view taken along the line E-E.

As shown in Fig. 1, a guiding catheter 1 is configured such that a shaft main body 10 is connected to a connector 2 through a protection shaft 3 mounted to the proximal end portion side of the shaft main body 10.

The shaft main body 10 includes a distal end portion 11, a first curved portion 12 connected to the distal end portion 11, a second curved portion 14, and a main body 15. The distal end portion 11 is a flexible straight tube member and has a flexible distal tip 4 formed at the distal end portion. Accordingly, the distal end portion 11 can be inserted from a patient's blood vessel inlet to a position around a treated part, for instance, a coronary artery inlet of a heart.

Accordingly, an outer diameter of the shaft main body 10 inserted into a patent's blood vessel is, for instance, in the range of 1.5 to 4.0 mm, and desirably in the range of 1.7 to 2.2 mm so as to be inserted into the radial artery. A length of the shaft main body 10 is in the range of 700 to 1,200 mm, and desirably in the range of 800 to 1,100 mm. In addition, an inner diameter of the shaft main body 10 is, for instance, in the range of 1.2 to 3.5 mm, and desirably in the range of 1.5 to 1.9 mm so as to insert a therapeutic device such as a balloon catheter thereinto in a state that a balloon portion is folded.

The curved portion including the first curved portion 12 and the second curved portion 14 which are continuously curved in the same direction allows the guiding catheter 1 to correspond to a blood vessel shape of the coronary artery. A position and a shape thereof are determined so as to correspond to a shape of the blood vessel of the coronary artery around the actual heart. The curved shape can be formed by, for instance, thermal deformation.

Next, a configuration of the shaft main body 10 will be described with reference to Fig. 4. As shown in Figs. 4A and 4B, the distal end portion 11, the first curved portion 12, the second curved portion 14, and the main body 15 constituting the shaft main body 10 respectively include an inner layer tube (inner layer) A which integrally forms inner circumferential portions of the respective portions and members, an outer layer tube (outer layer) B which forms outer circumferential portions of the respective portions and members, and a metallic reinforcing member (blade) C which is fixed to the outer layer tube B in a mesh shape, the inner layer tube A being fixed to the corresponding outer layer tube B.

The outer layer tube B and the inner layer tube A are adjacent to each other in an axis center direction and are connected to each other by welding, adhering, or integral forming process. The distal end portion 11, the first curved portion 12, the second curved portion 14, and the main body 15 constituting the shaft main body 10 communicate with one another in a full length of the shaft main body 10. In addition, in this embodiment, the respective inner layer tubes A are integrally formed with one another and the respective outer layer tubes B are integrally formed with one another. The elasticity becomes smaller in order from the main body, the curved portion, the distal end portion, and the distal tip.

The outer layer tube B is made from thermoplastic resin such as polyamide resin, polyether-based polyamide resin, polyester resin, or polyurethane resin which has a predetermined shape preservation property and elasticity. The inner layer tube A is made from fluorine-based resin such as polytetrafluoroethlene (PTFE) or tetrafluoroethlene-perfluoroalkylvinylether-copolymer (PFA) which has small friction coefficient in consideration of operability of a therapeutic device. In addition, contrast agents are mixed with the material of the outer layer in order to be visually recognized during a treatment. An example of the contrast agents includes bismuth oxide, bismuth subcarbonate, tungsten, or the like.

The distal tip 4 is connected to the distal end portion 11 of the shaft main body 10 by welding, adhering, or integral forming process so as to communicate with each other. As shown in Figs. 4A and 4B, the distal tip 4 is configured as a single layer tube and is made from material obtained by mixing contrast agents with thermoplastic resin. The resin and the contrast agents are the same as those of the outer layer tube B or flexible grade.

Although the curved portion may be formed into exemplary shapes such as a Judkins or an Amplatz or may be formed into other specific shapes, the Judkins is widely used in that operability is excellent. A right Judkins guiding catheter is used for an insertion operation into the right coronary artery. However, although the right Judkins guiding catheter has excellent operability in that the insertion operation into the coronary artery is easy, the right Judkins guiding catheter cannot strongly withstand a repulsive force occurring when a therapeutic device passes through a narrowed portion. As a result, the right Judkins guiding catheter comparatively easily comes off from the coronary artery. When the guiding catheter comes off from the coronary artery, the therapeutic device cannot pass through the narrowed portion any more. In addition, although an Amplatz guiding catheter can strongly withstand the repulsive force, an insertion operation into the coronary artery may be difficult or the coronary artery inlet may be damaged.

In this embodiment, the curved portion has a shape shown in Fig. 1 and includes the first curved portion 12 on the distal end portion side and the second curved portion 14 on the proximal end portion side. At this time, the first curved portion 12 and the second curved portion 14 are continuously connected to each other through the connection portion 13 and are curved in the same direction.

In addition, a curvature radius R2 of the second curved portion 14 is larger than a curvature radius R1 of the first curved portion 12. For instance, when the curvature radius R1 of the first curved portion 12 is in the range of 5 to 20 mm, the curvature radius R2 of the second curved portion 14 is in the range of 30 to 100 mm.

Such a guiding catheter 1 for the coronary artery having the shaft main body 10 with the above-described configuration can be used in a trans-radial coronary intervention (TRI) in which a small-diameter guiding catheter has to be used.

For instance, as shown in Fig. 2, the guiding catheter 1 is inserted into one of left and right radial arteries 31 which are close to a skin and then, the distal end portion 11 is sent to the right coronary artery so as to be latched therein. The radial artery 31 corresponds to one of two arteries branched from a brachial artery 30 and is connected to the other ulnar artery 32 through a palmar arch 33 positioned at a wrist 41. Accordingly, even in a closed state that the guiding catheter 1 is inserted into the radial artery 31, a critical aftereffect does not occur in the clinical examination due to the closed state of the radial artery 31 because a blood in the ulnar artery 32 continues to flow. In addition, it is possible to prevent the important arteries or nerves from being damaged upon inserting the guiding catheter 1 into the radial artery 31 because the radial artery 31 is sufficiently away from the important arteries or nerves.

Since the radial artery 31 is close to the skin, it is possible to easily control bleeding after the treatment and thus to reduce an occurrence risk of a bleeding complication. After the treatment using the guiding catheter 1 inserted from the radial artery, pressure is applied to the wound part for 15 minutes or so. Accordingly, a patient can be up and walk within a short time after the treatment, and thus the PCI is suitable for a patient. On the contrary, in a femoral catheter, a patient is restrained for about 4 to 6 hours after the treatment with pressure applied to the wound part to ensure that the bleeding stopped.

In the a trans-radial coronary intervention (TRI), for instance, the inside of the radial artery 31 of a right hand 40 of a patient 42 is punctured, and then, a sheath (not shown) is inserted from the punctured position into the inside of the artery. The distal end portion 11 of the guiding catheter 1 is first into the sheath and then is inserted into the radial artery 31. Subsequently, the distal end portion 11 is inserted from the radial artery 31 to an ascending aorta 21 through the brachial artery 30.

In order to assist an insertion operation in which the guiding catheter 1 is inserted from the radial artery 31 to the ascending aorta 21 of the heart 20, it is possible to straighten the curved portion on the distal end portion side in such a manner that a general guide wire (not shown) is inserted into a center hole of the catheter. The guide wire can be separated from the guiding catheter 1 after inserting the distal end portion 11 into the ascending aorta 21.

The distal end portion side of the guiding catheter 1 separated from the guide wire exhibits a predetermined curve shape. Since the guiding catheter 1 includes the first curved portion on the distal end portion side with a small curvature radius R1 and the second curved portion on the proximal end portion side with a large curvature radius R2, the guiding catheter 1 can be displaced to a position (Guiding catheter 1A) in which the guiding catheter 1 engages with a right coronary artery 22 from the right radial artery and a position (Guiding catheter 1B) in which the guiding catheter 1 engages with the right coronary artery 22 from the left radial artery.

That is, the distal end portion 11 having the distal tip 4 is inserted along one of left and right radial arteries to thereby reach the right coronary artery 22. Subsequently, a part of the curved portion comes into contact with an aortic wall 24 in terms of the second curved portion 14 having a predetermined appropriate curvature radius R2. Subsequently, the distal tip 4 on the linear distal end portion side is latched in the right coronary artery 22 in terms of the first curved portion having a predetermined appropriate curvature radius R1. As a result, the guiding catheter 1 hardly comes off from the right coronary artery 22.

Next, the respective cases will be described with reference to Figs. 3A and 3B.

Fig. 3A shows the guiding catheter 1A in which the distal end portion 11 is inserted into the right coronary artery 22 via a right radial artery approach. The guiding catheter 1A corresponds to a case in which the distal tip 4 is inserted into the right coronary artery 22 while the second curved portion 14 comes into contact with the aortic wall 24 as an intermediate wall of the ascending aorta 21. In this state, even in a case where a large repulsive force occurs when a therapeutic device such as a balloon catheter advances in a narrowed portion, the guiding catheter 1 can satisfactorily withstand the large repulsive force, thereby surely inserting the therapeutic device into the narrowed portion.

Fig. 3B shows the guiding catheter 1B in which the distal end portion 11 is inserted into the right coronary artery 22 via a left radial artery approach. In this case, the guiding catheter 1B corresponds to a case that the distal tip 4 is inserted into the right coronary artery 22 while the second curved portion 14 having a large curvature radius comes into contact with the aortic wall 24. Accordingly, even in a case where the large repulsive force occurs when the therapeutic device advances in a narrowed portion of the right coronary artery 22, the guiding catheter 1 can satisfactorily withstand the large repulsive force, thereby surely inserting the therapeutic device into the narrowed portion.

Upon enlarging the narrowed portion of the coronary artery of the heart, a guide wire for a guiding catheter is first inserted into an artery blood vessel in terms of a Seldinger technique or the like. Subsequently, the guiding catheter is inserted into the artery blood vessel along the guide wire so that the distal tip 4 is positioned at a coronary artery inlet of the heart.

Subsequently, only the guide wire is taken out therefrom and then the distal tip 4 of the guiding catheter 1 is inserted into the coronary artery. Subsequently, a thin guide wire for a therapeutic device thinner than the guiding catheter is inserted along the guiding catheter 1 so that the distal end portion arrives at a position past the narrowed portion. Subsequently, the distal end portion of the therapeutic device such as a balloon catheter is inserted into a proximal end portion of the guide wire, the therapeutic device such as the balloon catheter is inserted into the guiding catheter along the guide wire as a shaft, and then the distal end portion of the therapeutic device advances in the narrowed portion. At this time, the therapeutic device is applied with a repulsive force generated from the narrowed portion of the coronary artery.

In this case, when the repulsive force is small because a gap between the narrowed portion and the guide wire for the therapeutic device is large due to weakness of the narrowed portion, when the narrowed portion is flexible and the repulsive force is small, or when an outer diameter of the guiding catheter is large to carry out PTCA via a trans-femoral artery intervention (TFI) and a thickness is large, even the known guiding catheter not having a special technique in the curved portion can withstand the repulsive force while the curved portion comes into contact with the inner wall of the blood vessel. Accordingly, it is possible to satisfactorily advance the therapeutic device in the narrowed portion.

However, when the repulsive force occurring upon advancing the therapeutic device in the narrowed portion is large because the gap between the narrowed portion and the guide wire for the therapeutic device is small due to strength of the stenosis of the narrowed portion, when the narrowed portion is stiff and the repulsive force is large, or when the outer diameter of the guiding catheter is small to carry out PTCA via a trans-radial coronary intervention (TRI) for alleviating a patient's burden after the treatment, and the thickness is small and the inner diameter is large to carry out a treatment in which a comparatively large-diameter therapeutic device is inserted into the guiding catheter, the curved portion of the known guiding catheter not having a special technique in the curved portion is straightened by the repulsive force, and thus, the distal tip of the guiding catheter may come off from the inlet of the coronary artery in some cases. In this case, since the guiding catheter cannot withstand the repulsive force, it is difficult to advance the therapeutic device in the narrowed portion.

However, in this invention, the shape of the curved portion of the guiding catheter 1 is formed by the first curved portion 12 on the distal end portion side and the second curved portion 14 on the proximal end portion side. At this time, the first curved portion 12 and the second curved portion 14 are connected to each other through the connection portion 13 and are curved in the same direction. In addition, the curvature radius R2 of the second curved portion 14 is larger than the curvature radius R1 of the first curved portion 12. For instance, when the curvature radius R1 of the first curved portion 12 is in the range of 5 to 20 mm, the curvature radius R2 of the second curved portion 14 is in the range of 30 to 100 mm.

With such a configuration, it is possible to obtain the guiding catheter for the coronary artery capable of being easily inserted into the coronary artery even when a thickness of the guiding catheter is small for the a trans-radial coronary intervention, and of withstanding the repulsive force occurring upon inserting the therapeutic device into the narrowed portion with an excellent back-up force.

Next, a test for measuring a back-up force will be described with reference to a specific example and a comparative example of guiding catheters having curved portions with different shapes.

Example A: a tube was formed in such a manner that an inner layer tube A of a shaft main body was made from PTFE, an outer layer tube B thereof was made from nylon resin (where a distal end portion was made from flexible polyether block polyamide), a metallic reinforcing member (blade) C was filled between the inner and outer layer tubes in a mesh shape, and bismuth oxide as contrast agents was mixed with the resin of the outer layer, where the shapes of the distal end portion and curved portion were formed in the same manner as the guiding catheter shown Fig. 1, the guiding catheter including a second curved portion having a large curvature radius (for instance, 50 mm) and a first curved portion having a small curvature radius (for instance, 7 mm) continuously curved in the same direction.

Comparative Example 1: a tube was made from the same material described above, where the shape of the distal end portion was formed as a general right Judkins.

The test for the back-up force was carried out by using Example A and Comparative Example 1.

A push pull gauge was positioned at a right coronary artery inlet of an artificial blood vessel model prepared in warm water at 37°C. Subsequently, the guiding catheter was advanced from a position corresponding to left and right radial artery approach. Subsequently, a distal tip was strongly pushed into a distal end portion of the push pull gauge. Subsequently, a PTCA balloon catheter was moved along the inside of the guiding catheter at a constant speed. Finally, a load was measured at the time the distal tip of the guiding catheter come off from the push pull gauge. The result is shown in Table 1.

**Table 1**

| | Example A | Comparative Example 1 |
|---|---|---|
| Right radial artery approach | 8.2 g | 5.7 g |
| Left radial artery approach | 13.9 g | 11.9 g |

As clearly shown in Table 1, it proves that the Example A has larger load than the Comparative Example 1 at the time the distal tip comes off from the push pull gauge. That is, this test result shows that the Example A has an excellent back-up force for withstanding the repulsive force occurring upon inserting the therapeutic device into the narrowed portion.

According to the invention, since the curved portion provided between the distal end portion and the main body includes the first curved portion on the distal end portion side and the second curved portion on the proximal end portion side which are continuously curved in the same direction, it is possible to obtain the guiding catheter having an excellent back-up force by forming the curved portion with a shape having two different curvature radiuses so as to correspond to a lumen shape of a part into which the guiding catheter is inserted.

Further, since a portion from the first curved portion to the distal end portion is formed into a linear shape, it is possible to easily latch a short linear distal end portion in the coronary artery inlet of the ascending aorta. Furthermore, since the therapeutic device such as the balloon catheter can be advanced from the linear distal end portion in a linear shape, it is possible to accurately insert the therapeutic device into a predetermined diseased part. Accordingly, it is possible to obtain the guiding catheter for the coronary artery which is easily inserted into the coronary artery even via the trans-radial coronary intervention using a small-diameter guiding catheter and hardly comes off from the coronary artery thanks to a strong pushing force.

## Claims

1. A guiding catheter (1) comprising:
a distal end portion (11);
a main body (10); and
a curved portion which is provided between the distal end portion (11) and the main body (10),
wherein the curved portion includes a first curved portion (12) on a distal end portion side and a second curved portion (14) on a proximal end portion side, and
wherein the first curved portion (12) and the second curved portion (14) are continuously curved in the same direction, **characterized in that** the catheter is a trans-radial coronary intervention catheter, wherein the curvature radius of the first curved portion (12) is in the range of 5 to 20 mm and the curvature radius of the second curved portion (14) is in the range of 30 to 100 mm.

2. The guiding catheter (1) according to Claim 1,
wherein a curvature radius of the second curved portion (14) is larger than that of the first curved portion (12).

3. The guiding catheter (1) according to Claim 1 or 2,
wherein a portion from the first curved portion (12) to the distal end portion (11) is formed into a linear shape.

4. The guiding catheter (1) according to any one of Claims 1 to 3,
wherein the distal end portion (11) is formed into a linear shape.

5. The guiding catheter (1) according to any one of Claims 1 to 4,
wherein an elastic distal tip (4) is provided at the end of the distal end portion (11).

6. The guiding catheter (1) according to Claims 4 and 5,
wherein the guiding catheter (1) is configured such that the distal tip (4) is insertable from one of left and right radial arteries (31) to a right coronary artery (22) so that a part of the curved portion comes into contact with an aortic wall (24) using the second curved portion (14) having a predetermined curvature radius and the distal tip (4) positioned at the linear shaped distal end portion (11) is latched in the right coronary artery (22) using the first curved portion (12) having a predetermined curvature radius so as not to come off from the right coronary artery (22).

## Patentansprüche

1. Führungs-Katheter (1), umfassend:
einen distalen Endabschnitt (11);
einen Hauptkörper (10); und
einen gebogenen Abschnitt, welcher zwischen dem distalen Endabschnitt (11) und dem Hauptkörper (10) angeordnet ist,
wobei der gebogene Abschnitt einen ersten gebogenen Abschnitt (12) auf einer distalen Endabschnittsseite und einen zweiten gebogenen Abschnitt (14) auf einer proximalen Endabschnittsseite umfasst, und
wobei der erste gebogene Abschnitt (12) und der zweite gebogene Abschnitt (14) kontinuierlich in derselben Richtung gebogen sind,
**dadurch gekennzeichnet,**
**dass** der Katheter ein trans-radialer koronarer Eingriffs-Katheter ist, wobei der Krümmungsradius des ersten gebogenen Abschnitts (12) in dem Bereich von 5 - 20 mm und der Krümmungsradius des zweiten gebogenen Abschnitts (14) in dem Bereich von 30 - 100 mm liegt.

2. Führungs-Katheter (1) nach Anspruch 1,
**dadurch gekennzeichnet,**
**dass** ein Krümmungsradius des zweiten gebogenen Abschnitts (14) größer als derjenige des ersten gebogenen Abschnitts (12) ist.

3. Führungs-Katheter (1) nach Anspruch 1 oder 2,
**dadurch gekennzeichnet,**
**dass** ein Abschnitt von dem ersten gebogenen Abschnitt (12) bis zu dem distalen Endabschnitt (11) in einer geradlinigen Form ausgebildet ist.

4. Führungs-Katheter (1) nach einem der Ansprüche 1 bis 3,
**dadurch gekennzeichnet,**
**dass** der distale Endabschnitt (11) in einer geradlinigen Form ausgebildet ist.

5. Führungs-Katheter (1) nach einem der Ansprüche 1 bis 4,
**dadurch gekennzeichnet,**
**dass** eine elastische distale Spitze (4) an dem Ende des distalen Endabschnitts (11) vorhanden ist.

6. Führungs-Katheter (1) nach Anspruch 4 und 5,
**dadurch gekennzeichnet,**
**dass** der Führungs-Katheter (1) derart ausgestaltet ist, dass die distale Spitze (4) von der linken oder rechten Arteria radialis (31) in eine rechte Koronararterie (22) einführbar ist, so dass ein Teil des gebogenen Abschnitts in einen Kontakt mit einer Aorta-Wand (24) kommt, wobei der zweite gebogene Abschnitt (14) mit einem vorbestimmten Krümmungsradius verwendet wird und die distale Spitze (4), welche an dem geradlinig geformten distalen Endabschnitt (11) angeordnet ist, unter Verwendung des ersten gebogenen Abschnitts (12) mit einem vorbestimmten Krümmungsradius in der rechten Koronararterie (22) arretiert ist, so dass sie sich nicht von der rechten Koronararterie (22) ablöst.

## Revendications

1. Cathéter de guidage (1) comprenant :
une partie d'extrémité distale (11) ;
un corps principal (10) et
une partie incurvée qui est prévue entre la partie d'extrémité distale (11) et le corps principal (10),
dans lequel la partie incurvée comprend une première partie incurvée (12) sur un côté de partie d'extrémité distale et une seconde partie incurvée (14) sur un côté de partie d'extrémité proximale, et
dans lequel la première partie incurvée (12) et la seconde partie incurvée (14) sont incurvées de manière continue dans la même direction, **caractérisé en ce que** le cathéter est un cathéter d'intervention coronarienne par voie transradiale, dans lequel le rayon de courbure de la première partie incurvée (12) est de l'ordre de 5 à 20 mm et le rayon de courbure de la seconde partie incurvée (14) est de l'ordre de 30 à 100 mm.

2. Cathéter de guidage (1) selon la revendication 1,
dans lequel un rayon de courbure de la seconde partie incurvée (14) est supérieur à celui de la première partie incurvée (12).

3. Cathéter de guidage (1) selon la revendication 1 ou 2, dans lequel une partie, de la première partie incurvée (12) à la partie d'extrémité distale (11) est formée selon une forme linéaire.

4. Cathéter de guidage (1) selon l'une quelconque des revendications 1 à 3, dans lequel la partie d'extrémité distale (11) est formée selon une forme linéaire.

5. Cathéter de guidage (1) selon l'une quelconque des revendications 1 à 4, dans lequel une pointe distale élastique (4) est prévue au niveau de l'extrémité de la partie d'extrémité distale (11).

6. Cathéter de guidage (1) selon les revendications 4 et 5, dans lequel le cathéter de guidage (1) est configuré de sorte que la pointe distale (4) peut s'insérer à partir d'une artère parmi les artères radiales gauche et droite (31) dans une artère coronaire droite (22) de sorte qu'une partie de la partie incurvée vient en contact avec une paroi aortique (24) en utilisant la seconde partie incurvée (14) ayant un rayon de courbure prédéterminé et la pointe distale (4) positionnée au niveau de la partie d'extrémité distale (11) de forme linéaire est verrouillée dans l'artère coronaire droite (22) en utilisant la première partie incurvée (12) ayant un rayon de courbure prédéterminé de sorte qu'elle ne sort pas de l'artère coronaire droite (22).
